# EUROPEAN PATENT SPECIFICATION

(11) **EP 3 250 397 B1**
(45) Date of publication and mention of the grant of the patent: **27.03.2019**
(21) Application number: 16703931.2
(22) Date of filing: 25.01.2016
(51) Int. Cl.: B60C 1/00, C08C 19/06, C08C 19/20, C08L 9/00, C08L 9/06

(54) **FUNCTIONALIZED ELASTOMER, METHOD OF MAKING, AND USES THEREOF**
FUNKTIONALISIERTES ELASTOMER, VERFAHREN ZUR HERSTELLUNG UND VERWENDUNGEN DAVON
ÉLASTOMÈRE FONCTIONNALISÉ, PROCÉDÉ DE FABRICATION, ET UTILISATIONS ASSOCIÉES

(30) Priority: 28.01.2015 US 201562108775 P
(43) Date of publication of application: 06.12.2017
(73) Proprietor: SABIC Global Technologies B.V., 4612 PX Bergen op Zoom (NL)
(72) Inventor: NANDY, Ritesh, Bangalore 562 125 Karnataka (IN); PATIL, Prashant, Bangalore 562 125 Karnatak (IN); MITRA, Susanta, Bangalore 562 125 Karnataka (IN); HAN, Seung, 11422 Riyadh (SA); HAN, Seungcheol, 6160 GA Geleen (NL)
(74) Representative: Sabic Intellectual Property Group
(86) International application number: PCT/EP2016/051446
(87) International publication number: WO 2016/120206

(56) References cited:
- EP-A1- 2 289 712
- WO-A1-2014/108958
- WO-A2-2007/024797
- DATABASE WPI Week 200166 Thomson Scientific, London, GB; AN 2001-586230 XP002757248, & JP 2001 172499 A (DAICEL CHEM IND LTD) 26 June 2001 (2001-06-26)

## Description

### BACKGROUND

This disclosure relates to functionalized elastomers that include epoxy, hydroxy, and thio functional groups, methods of preparing the functionalized elastomer, and uses thereof.

Approximately 20-30% of a vehicle's fuel consumption relates to its tires. Vehicular tires have a tendency to resist rolling while in operation, due at least in part to adhesion of the tire's rubber to the road it is rolling on. Vehicles overcome the rolling resistance of tires by producing more energy, thereby burning more fuel. Low rolling resistance tires require less energy as the tire rolls, thereby increasing a vehicle's fuel efficiency. Reducing rolling resistance in tires is therefore desirable for cars configured to meet today's increasingly demanding standards for lower fuel consumption and reduced carbon dioxide emission.

Tire manufacturers have taken a number of approaches to developing and manufacturing low rolling resistance tires. For example, incorporation of functionalized elastomers into vehicular rubber tires can improve tire performance properties. The functional groups can act to modify the interaction of the elastomer with the fillers that are also present in tires, primarily silica. Functionalization of elastomers can be achieved either during or after polymerization. Functionalization during polymerization uses functional monomers, initiators, or termination agents. Functionalization during polymerization, i.e., co-polymerization and/or end-chain polymerization, has several known disadvantages. For example, copolymerization often requires complex syntheses to make the functional monomers. A further limitation is the choice of suitable functional groups for functionalization during polymerization, as the functional groups are limited to those that do not react with the polymerization initiator. End-chain functionalization can be accomplished, but due to the relatively high molecular weight of the tire rubber, the weight ratio of end groups to main chain can be too low, so that the end groups do not significantly impact the elastomer-filler interaction.

Post-polymerization functionalization can be achieved by a variety of methods. For example, silane coupling agents can be used in conjunction with silica fillers. Use of the silanes, however, can lead to an undesirable increase in composition viscosity during tire manufacturing.

There remains a need in the art for the post-polymerization functionalization of elastomers, in particular, methods that provide useful functional groups for modifying the properties of the elastomers when used in tires.

### SUMMARY OF THE INVENTION

The above and other deficiencies are overcome by, in an embodiment, methods of preparing a functionalized elastomer, the method comprising epoxy ring-opening an epoxidized elastomer with a C₁-C₃₂ hydrocarbyl-substituted thiol to provide a functionalized elastomer comprising an epoxy functional group, a hydroxy functional group, and a substituted or unsubstituted C₁-C₃₂ hydrocarbyl-substituted thio functional group, wherein the hydroxy functional group and the substituted or unsubstituted C₁-C₃₂ hydrocarbyl-substituted thio functional group are vicinal functional groups.

Also disclosed herein are functionalized elastomers prepared by the methods.

In another embodiment, a functionalized elastomer comprises an epoxy functional group, a hydroxy functional group, and a C₁-C₃₂ hydrocarbyl-substituted thio functional group, wherein the hydroxy functional group and the C₁-C₃₂ hydrocarbyl-substituted thio functional group are vicinal functional groups.

Articles comprising the functionalized elastomers are also disclosed, in particular tires comprising the functionalized elastomers.

### BRIEF DESCRIPTION OF THE DRAWINGS

FIG. 1 illustrates a proton nuclear magnetic resonance (¹H-NMR) spectrum for an epoxidized polybutadiene rubber (PBR) elastomer obtained in accordance with Example 1.
FIG. 2 shows a ¹H-NMR spectrum for a functionalized PBR elastomer obtained according to Example 2.
FIG. 3A and 3B illustrate an infrared (IR) spectrum for propane thiol, an unsaturated PBR elastomer, an epoxidized PBR elastomer and a functionalized PBR elastomer obtained in Example 2.
FIG. 4 shows the effect of time and epoxidized elastomer concentration during epoxy ring-opening. In the graph, percent (%) functionalization is plotted over time in hours (h) for two different PBR concentrations (2% and 4%) in THF solvent.

### DETAILED DESCRIPTION OF THE INVENTION

The inventors hereof have discovered methods for the post-polymerization functionalization of elastomers, in particular methods for providing functionalities that are difficult to obtain by copolymerization. In the methods, an unsaturated elastomer is epoxidized, then subjected to epoxy ring-opening with a substituted thiol. The elastomers functionalized by this method include epoxy functional groups, hydroxy functional groups, and substituted thio functional group. The hydroxy functional group and the substituted thio functional group are on adjacent carbon atoms, i.e., they are vicinal. Unsaturated groups can also be present. The functionalized elastomers have a variety of uses, including in vehicular tires.

In a key step of the method, an epoxidized elastomer is reacted with a substituted thiol to ring-open a portion of the epoxy groups. Thus, in an embodiment, a method of preparing the functionalized elastomer includes epoxy ring-opening an epoxidized elastomer with a C₁-C₃₂ hydrocarbyl-substituted thiol. Epoxidized elastomers can be obtained commercially, or synthesized as part of the methods described herein. Thus, in another embodiment, a method of preparing the functionalized elastomer includes epoxidizing an unsaturated elastomer to provide an epoxidized elastomer; and epoxy ring-opening the epoxidized elastomer with a C₁-C₃₂ hydrocarbyl-substituted thiol.

Methods for epoxidizing elastomers are known, and include, for example, peroxide-mediated epoxidation of an unsaturated elastomer. Epoxidized elastomers contain an epoxy (oxirane) functional group as a result of epoxidation. Elastomers, also known as rubbers, are amorphous viscoelastic polymers. Unsaturated elastomers have a plurality of carbon-carbon double bonds. The degree of unsaturation can be expressed in mole percent (mol%) unsaturation. For example, the elastomers can have a degree of unsaturation of at least 0.1 mol%, at least 0.5 mol%, at least 1 mol%, at least 2 mol% or at least 5 mol%, up to 80 mol%. For example, the degree of unsaturation can be 0.1 to 60 mole%, or 0.1 to 50 mol %, or 0.1 to 40 mol%.

The unsaturated elastomers can be homopolymers or copolymers, uncrosslinked, crosslinked, vulcanized, (e.g., vulcanized rubber) or the like, with unsaturation in the backbone or in side chains. The elastomers can have a glass transition temperature (Tg) below 20°C, or below 0°C, for example from -110°C to -20°C or -90°C to -10°C, as determined according to ASTM D7426-08 (2013). The elastomers can be of natural origin or synthetic, and can be obtained, for example, by solution polymerization, emulsion polymerization or gas-phase polymerization of one or more conjugated diolefins, optionally combined with one or more ethylenically unsaturated comonomers such as monovinyl arenes, (C₁-C₆ alkyl) (meth)acrylates, and the like.

Examples of unsaturated elastomers include natural polyisoprene rubber, a synthetic polyisoprene rubber, a homopolymer of 1,3-butadiene, 2-methyl-1,3-butadiene, 1,3-pentadiene, 2-chloro-1,3 butadiene, or 2,3-dimethyl-1,3-butadiene, a copolymer of 1,3-butadiene, 2-methyl-1,3-butadiene, 1,3-pentadiene, 2-chloro-1,3 butadiene, or 2,3-dimethyl-1,3-butadiene with styrene, alpha-methylstyrene, acrylonitrile, isoprene, methacrylonitrile, methyl acrylate, ethyl acrylate, methyl methacrylate, ethyl methacrylate, or vinyl acetate, ethylene-vinyl acetate, chloroprene rubber, butyl rubber, halogenated butyl rubber, nitrile rubber, hydrogenated nitrile rubber, ethylene-propylene rubber, ethylene-propylene-diene rubber, unsaturated silicone rubber, or a combination comprising at least one of the foregoing.

Specific elastomers include polybutadiene rubber, poly(acrylonitrilebutadiene), poly(styrene-butadiene) rubber, polyisoprene rubber, neoprene rubber, preferably polybutadiene rubber, poly(styrene-butadiene) rubber, or a combination comprising at least one of the foregoing.

Epoxidation of the unsaturated elastomers can be effected by the use of epoxidizers. Examples of epoxidizers include peroxides such as peroxyacids, peracids, inorganic peroxyacids, organic peroxy acids, and the like. Preferred inorganic peroxyacids are peroxymonosulfuric acid, peroxyphosphoric acid, perboric acid, perborates, and the like. Among the organic peroxy acids, peracetic acid, peroxybenzoic acid, or m-chloroperoxybenzoic acid (m-CPBA) can be used.

The quantity of the peroxide in the epoxidation is selected to achieve the desired degree of epoxidation, and can be determined by one of ordinary skill in the art without undue experimentation. For example the amount of peroxide can be between 5 and 100 weight percent (wt%) based on the weight of the unsaturated elastomer, or can be 20 to 90 wt%, or 30 to 80 wt%, or 40 to 80 wt%, based on the weight of the unsaturated elastomer.

Preferably, the epoxidation is carried out in the presence of a solvent. The solvent, if used, is selected to dissolve the unsaturated elastomer. Examples of suitable solvents include aliphatic, cycloaliphatic, halogenated aliphatic or cycloaliphatic, heterocycloaliphatic, aromatic, heteroaromatic solvents, for example, dichloromethane, chloroform, carbon tetrachloride, pentane, n-hexane, cyclopentane, cyclohexane, n-heptane, benzene, toluene, xylene including o-,m-, p-xylene, cumene, tetrahydrofuran, dioxane, pyridine, or a combination comprising at least one of the foregoing.

If a solvent is used, the elastomer can have a concentration in the solvent of 1 to 22% (weight/volume (w/v)), 1 to 20% (w/v), 1 to 18% (w/v), 2 to 16 % (w/v), 2 to 12% (w/v), preferably 2 to 10% (w/v), more preferably 2 to 8% (w/v). Of course, other concentrations can be used.

In an aspect of the method, the unsaturated elastomer is dissolved in the solvent to provide a solution, and the peroxide is added to the solution to epoxidize a portion of the unsaturated groups (carbon-carbon double bonds) in the elastomers. Other orders of addition can be used. Conditions for epoxidation can be determined without undue experimentation, for example, a temperature of 0 to 100°C, preferably 20 to 80°C, or 20 to 40°C, for 1 min to 6 hours, or 1 to 6 hours, alternatively 1 min to 3 hours, or 1 to 3 hours, at atmospheric pressure.

Optionally, the reaction mixture is quenched to neutralize acids and terminate the reaction. A base can be used for quenching, for example an alkali or alkaline earth carbonate, bicarbonate, oxide, or hydroxide, for example, an aqueous solution of sodium carbonate, or of sodium bicarbonate, or an alcoholic, e.g., methanolic, solution of tetrabutylammonium hydroxide, potassium hydroxide, or the like. Quenching can result in the formation of an aqueous phase and an organic phase, which can be separated, and the aqueous phase discarded after separation. Isolation of the epoxidized elastomer can be by precipitation with a nonsolvent such as methanol.

Preferably, not all of the unsaturated groups are reacted during elastomer functionalization, such that double bonds remain in the functionalized elastomer. The epoxidized elastomers can have a degree of epoxidation of 1 to 50 mol%, 1 to 40 mol%, 1 to 30 mol%, 2 to 40 mol%, 2 to 30 mol%, or 3 to 30 mol%.

The epoxidized elastomer is reacted with a substituted thiol to epoxy ring-open a portion of the epoxy groups and provide vicinal hydroxy and substituted thio groups. "Vicinal" as used herein means that the hydroxy and the substituted thio group arising from each ring opening are bonded to two different, adjacent carbon atoms.

The substituted thiol is a C₁-C₃₂ hydrocarbyl-substituted thiol, where the C₁-C₃₂ hydrocarbyl can be a substituted or unsubstituted C₁-C₃₂ alkyl group, a substituted or unsubstituted C₂-C₃₂ alkenyl group, a substituted or unsubstituted C₃-C₁₈ cycloalkyl group, a substituted or unsubstituted C₃-C₁₈ heterocycloalkyl group, a substituted or unsubstituted C₆-C₁₈ aryl group, or a substituted or unsubstituted C₄-C₁₈ heteroaryl group.

Preferably the C₁-C₃₂ hydrocarbyl-substituted thiol group is a substituted or unsubstituted C₁-C₁₆ alkyl group, a substituted or unsubstituted C₃-C₁₆ cycloalkyl group, a substituted or unsubstituted C₃-C₁₆ heterocycloalkyl group, a substituted or unsubstituted C₆-C₁₆ aryl group, a substituted or unsubstituted C₄-C₁₆ heteroaryl group, or a combination comprising at least one of the foregoing.

In a preferred embodiment, the C₁-C₃₂ hydrocarbyl-substituted thiol group is a substituted or unsubstituted C₁-C₁₂ alkyl group, more preferably an unsubstituted C₂-C₈ alkyl group, most preferably an unsubstituted C₃-C₈ alkyl group.

The substituted thiol may for example be selected from 1-propanethiol, methyl-3-mercaptopropionate, 1-butanethiol, 2-butanethiol, 2-methyl-1-propanethiol, 2-methyl-2-propanethiol, 4-cyano-1-butanethiol, 1-pentanethiol, 3-methyl-1-butanethiol, 1-hexanethiol, butyl-3-mercaptopropionate, 1-heptanethiol, 1-octanethiol, 2-ethylhexanethiol, 1-nonanethiol, tert-nonyl mercaptan, 1-decanethiol, 1-undecanethiol, 1-dodecanethiol, tert-docecylmercaptan, 1-tetradecanethiol, 1-pentadecanethiol, 1-hexadecanethiol, cis-9-octadecene-1-thiol, 1-octadecenethiol, cyclopentanethiol, cyclohexanethiol, 2-phenylethanethiol, phenylthiol, halogenated phenylthiol, nitrophenylthiol, methoxyphenylthiol, 2-propene-1-thiol, 2-furanemethanethiol, 2-methyl-3-furanethiol, 2-(trimethylsilyl)ethanethiol, 1-adamantanethiol, 2-propene-1-thiol, or combinations thereof. Preferably, the substituted thiol is selected from 1-propanethiol, 1-butanethiol, 2-methyl-1-propanethiol, 1-pentanethiol, 3-methyl-1-butanethiol, 1-hexanethiol, 1-heptanethiol, 1-octanethiol, 2-ethylhexanethiol, cyclopentanethiol, cyclohexanethiol, 2-phenylethanethiol, phenylthiol, 2-propene-1-thiol, 2-furanemethanethiol, or combinations thereof.

The epoxy ring-opening can be conducted in the presence of a solvent. If a solvent is present, the epoxidized elastomer is dissolved in the solvent. Examples of solvents are aliphatic, cycloaliphatic, halogenated aliphatic or cycloaliphatic, heterocycloaliphatic, aromatic, heteroaromatic solvents, preferably dichloromethane, chloroform, carbon tetrachloride, pentane, n-hexane, cyclopentane, cyclohexane, n-heptane, benzene, toluene, xylene including o-,m-, p-xylene, cumene, tetrahydrofuran, dioxane, or a combination comprising at least one of the foregoing.

The epoxidized elastomer can have a concentration in the solvent of 1 to 22% (w/v). For example, the epoxidized elastomer can have a concentration from 2 to 16% (w/v), 2 to 12% (w/v), 2 to 10% (w/v), 2 to 8% (w/v), or 2 to 6% (w/v).

The concentration of the substituted thiol in the solvent can be 5 to 70 wt%, 10 to 70 wt%, 5 to 60 wt%, 10 to 60 wt%, 15 to 60 wt%, or preferably 15 to 50 wt%, based on the weight of the epoxidized elastomer.

In some embodiments, the epoxy-ring opening is conducted in the presence of a catalyst capable of simultaneously opening the epoxy ring or promoting the addition of the substituted thiol. The catalyst can be acidic, for example perchloric acid, or a nucleophilic base. Exemplary bases include an alkali or alkaline earth oxide or hydroxide, such as sodium hydroxide, potassium hydroxide, magnesium hydroxide, or calcium hydroxide, a rare earth hydroxide such as lanthanum hydroxide or cerium hydroxide, an organic hydroxide such as a tetra(Ci-C6)alkyl ammonium hydroxide (e.g., tetrabutylammonium hydroxide, or an organic base such as a tertiary amine (e.g., 1,8- diazabicyclo[5.4.0]undec-7-ene, 1,4-diazabicyclo [2.2.2] octane, triethylamine, pyridine, or the like). A combination of different bases can be used. Preferably, the base is potassium hydroxide or tetrabutylammonium hydroxide. In some embodiments, the pH of the reaction mixture can be adjusted with the base. For example, the pH can be adjusted to a pH from 9 to 14, from 10 to 13, or from 10 to 12.

Preferably, the base is selected such that the base forms a homogeneous medium with the solvent used in epoxy ring-opening. If the solvent is immiscible or has low miscibility with the base, a heterogeneous mixture can form. If a heterogeneous mixture forms, epoxy ring-opening can be slow. Thus, in an embodiment, an alcoholic, e.g., a methanolic base solution is used. The alcoholic base solution can have a molarity of 0.1 to 10 molar (M) of the base in the alcohol. For example, the alcoholic solution can be from 0.2 to 10 M, 0.3 to 9 M, 0.5 to 8 M, 1 to 5 M, or 1 to 3 M. In an embodiment, epoxy ring-opening is conducted in THF as a solvent, with a methanolic base to provide a homogeneous medium.

The epoxy ring-opening is conducted at a temperature of 0 to 100°C, for example 20 to 80°C, preferably at 20 to 60°C. An effective time can be 1 hour to 5 days, depending on reactivity of the starting materials, temperature. Atmospheric pressure or higher can be used. Isolation can be by precipitation with a nonsolvent such as methanol. After the precipitation, the functionalized elastomer can be collected, for example by filtration.

Any of the foregoing processes can be carried out batch-wise or continuously. In an advantageous feature, the embodiment epoxidation of the unsaturated elastomer and epoxy ring-opening can be conducted without isolation of the intermediate epoxidized elastomer. Thus, in an embodiment, the method comprises elastomer epoxidation and epoxy ring-opening of epoxidized elastomer with a substituted thiol to provide the functionalized elastomers carried out in the same reactor or reaction vessel.

The functionalized elastomer can have a total hydroxy, substituted thio, and epoxy group functionalization of 1 to 15 mol%, 1 to 12 mol%, more preferably 1 to 10 mole%. In addition, the functionalized elastomer contains unsaturation. The relative ratios of each of the hydroxy, epoxy, thio, and unsaturated groups can be varied, by varying the epoxidation and epoxy ring-opening conditions, in order to adjust the properties of the functionalized elastomers. For example, based on the total number of the functionalized groups, the elastomer can comprise 1 to 30 % epoxy groups, 0.1 to 30 % hydroxyl groups, 0.1 to 30% substituted thio groups and 20 to 89.7% unsaturated groups, more specifically 1 to 20% epoxy groups, 0.1 to 20 % hydroxyl groups, 0.1 to 20% substituted thio groups and 20 to 80 % unsaturated groups.

The functionalized elastomers can be used in a wide variety of articles, for example in hoses, inner tubes, pads, solid rocket fuels, golf balls, encapsulation of electronic assemblies, footwear, wire and cable coatings, conveyer belts engine belts, tires and tire treads.

In a preferred embodiment the functionalized elastomers are a component of a tire tread for a vehicle, for example cars, trucks, and the like. Without being bound by theory, it is believed that the functional groups of the functionalized elastomer, in particular the hydroxy functional group, can modify the interaction between the elastomer and filler in a tire tread. Further, the loss of unsaturation due to functionalization can be compensated by the presence of the C₁-C₃₂ hydrocarbyl-substituted thio functional group, which can participate in crosslinking or vulcanization. The tire treads can accordingly have reduced rolling resistance.

The following Examples are provided for illustrative purposes only and are not to be construed as limiting in any manner.

### EXAMPLES

### Example 1. Elastomer Epoxidation

Polybutadiene rubber with a 1,4-cis content of 98 % (5 grams (g)) was dissolved in 100 milliliters (mL) of dichloromethane to achieve a 5% (w/v) elastomer solution. Next, 40 wt. % of m-chloroperoxybenzoic acid (m-CPBA) was added to the solution and the reaction mixture was stirred at room temperature for 3 h. Upon completion the reaction mixture was quenched with aqueous sodium carbonate (Na₂CO₃) or bicarbonate (NaHCO₃) solution. The organic and aqueous layers were separated in a separatory funnel. The organic layer was poured into methanol at a ratio of 1:2 (reaction mixture/methanol, volume/volume (v/v)) with stirring. A precipitate formed which was filtered and dried.

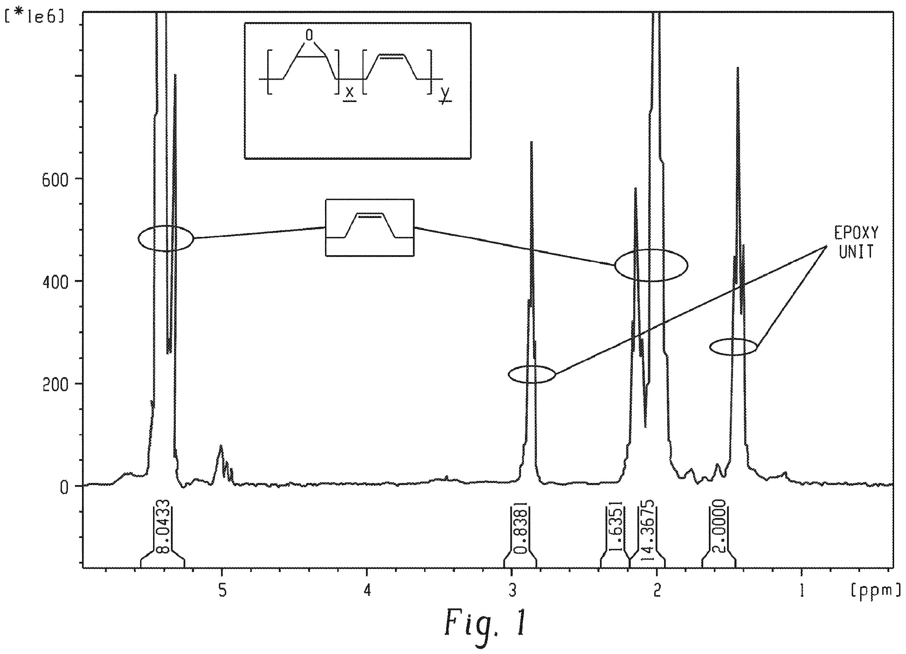

The product was characterized by ¹H NMR spectroscopy. As shown in FIG. 1, ¹H NMR spectroscopy analysis (300 MHz; in CDCl₃) showed that 9-10 mol% epoxidation of the elastomer was achieved.

### Example 2. Epoxy Ring-opening

A 2% (w/v) epoxidized elastomer solution was prepared in toluene. A 40 wt% (w/w) methanolic solution of 1-propanethiol and a 1 molar tetrabutyl ammonium hydroxide (TBAH) solution in methanol were added to the toluene solution. The reaction mixture was stirred at 23°C for 72 h and then poured into methanol. A precipitate formed which was filtered off and dried at 23°C. The filtered product was characterized by ¹H NMR and infrared (IR) spectroscopic methods.

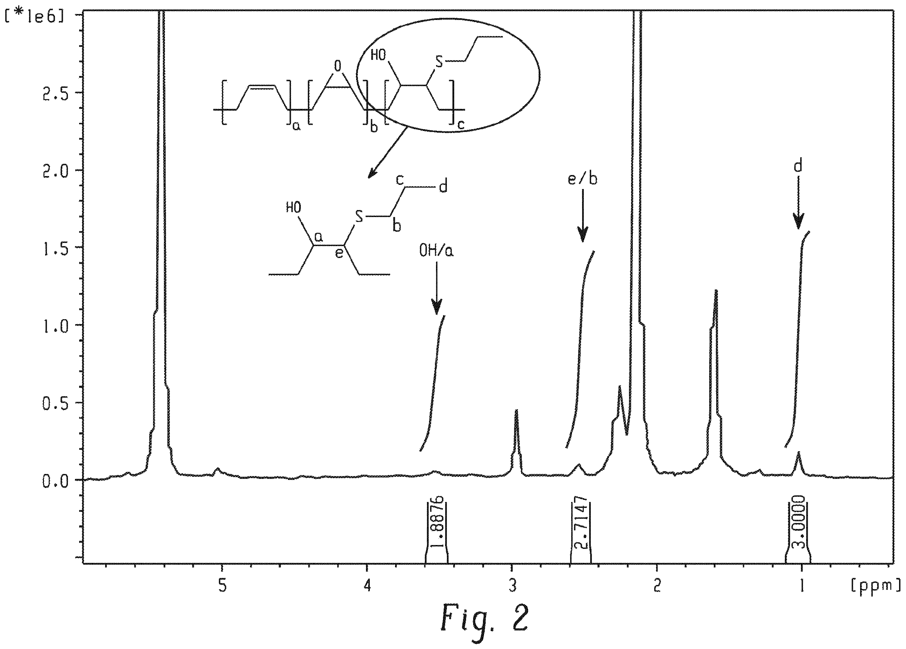

FIG. 2 shows the ¹H NMR analysis (300 MHz; in CDCl₃), which indicates 3% thio functionalization for the isolated functionalized elastomer. The functionalized elastomer comprises units as shown in formula (I) 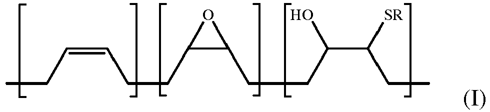 wherein R is n-propyl. Other units can be present in the elastomer, e.g., units arising from 1,2-addition.

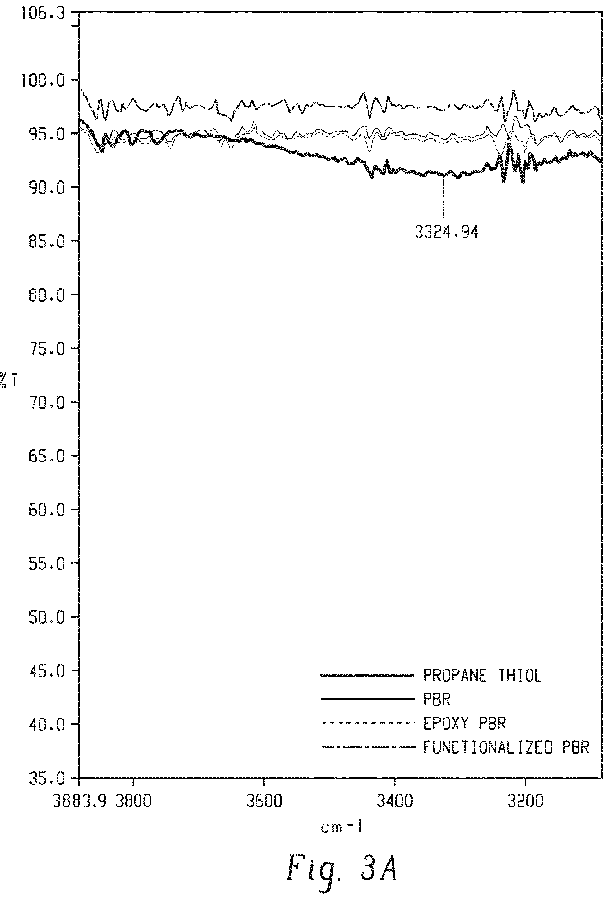

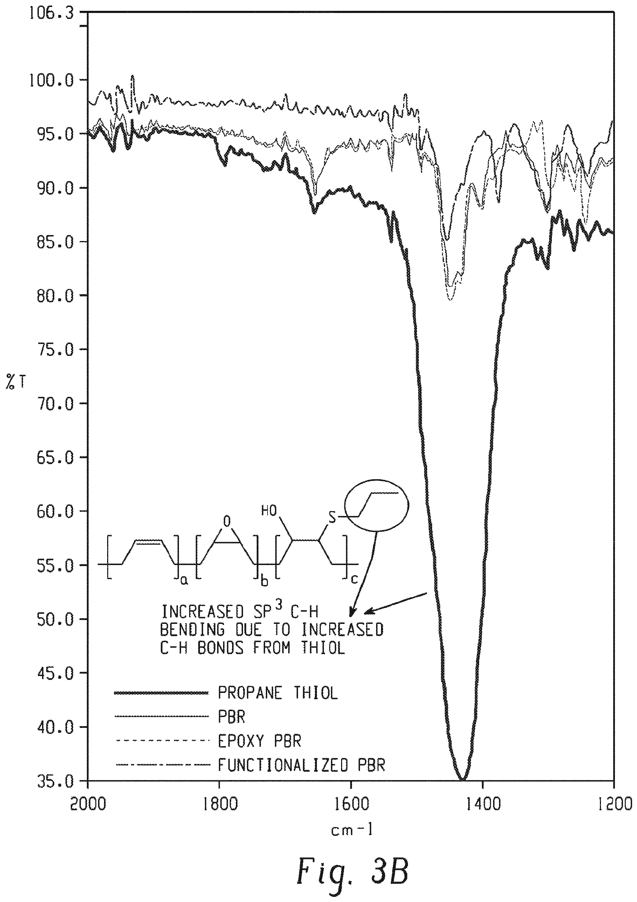

Comparative IR analysis (KBr pellet) of the polybutadiene rubber, the epoxidized polybutadiene of Example 1, and the functionalized elastomer of Example 2 confirms the presence of hydroxy functional groups in the functionalized elastomer . FIG. 3a shows that no hydroxy functional groups were present in the elastomer before or after epoxidation, but that hydroxyl groups are present in the elastomer after ring-opening. Fig. 3B illustrates that increased sp³ C-H bending is further observed in the functionalized elastomer, due the presence of the n-propyl groups in the thiol.

### Example 3: Effect of Time and Elastomer Concentration on Functionalization

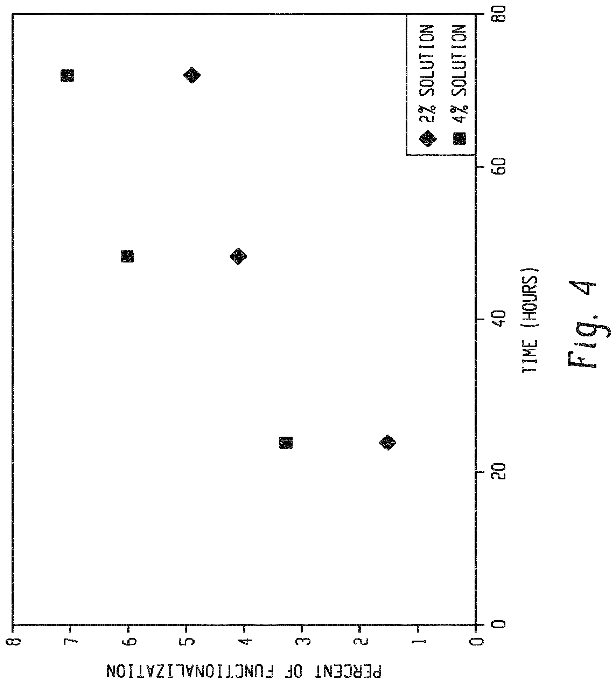

The procedure in Example 2 was followed except that both a 2% and 4% (w/v) epoxidized elastomer solution in THF solvent were prepared. A sample of the functionalized elastomer precipitate was characterized by ¹H NMR spectroscopic analysis at 24 h intervals. Percent functionalization found was then plotted over time elapsed, as shown in FIG. 4. The results from this testing show that with increasing concentration, the reaction becomes faster. The results also show that functionalization increases with time.

### Example 4. One-pot Synthesis of Functionalized Elastomer

A 5% (w/v) solution of the butadiene rubber used in Example 1 in tetrahydrofuran was prepared, then m-CPBA was added to the solution to achieve a concentration of 60% MCPBA/elastomer (w/w). The reaction mixture was stirred at 23°C for 3 h. The reaction mixture was made alkaline by adding a 1.5 molar TBAH solution in methanol. To this mixture 1-propanethiol was added to achieve a concentration of 30% propanethiol/elastomer (w/w). The reaction mixture was stirred at 50 to 55°C for 7 h, then the reaction mixture was brought to a temperature of 23°C and poured into methanol (1/2.5 of reaction mixture/methanol, v/v). A precipitate formed, which was isolated and dried at 23°C.

¹H NMR analysis (300 MHz; in CDCl₃) indicated functionalization as follows: 14 mole% epoxidation, where approximately 70 mol% of the epoxy groups had undergone epoxy ring-opening with propanethiol. Hence, 9 to 10% functionalization was achieved with respect to the propanethio functional group.

The methods and compositions are further illustrated by the following embodiments.
Embodiment 1: A method of preparing a functionalized elastomer, the method comprising: providing an epoxidized elastomer; epoxy ring-opening of the epoxidized elastomer with a C₁-C₃₂ hydrocarbyl-substituted thiol to provide a functionalized elastomer comprising: an epoxy functional group, a hydroxy functional group, and a substituted or unsubstituted C₁-C₃₂ hydrocarbyl-substituted thio functional group, wherein the hydroxy functional group and the substituted or unsubstituted C₁-C₃₂ hydrocarbyl-substituted thio functional group are vicinal functional groups.
Embodiment 2: The method of embodiment 1, wherein the hydrocarbyl group is a substituted or unsubstituted C₁-C₃₂ alkyl group, a substituted or unsubstituted C₂-C₃₂ alkenyl group, a substituted or unsubstituted C₃-C₁₈ cycloalkyl group, a substituted or unsubstituted C₃-C₁₈ heterocycloalkyl group, a substituted or unsubstituted C₆-C₁₈ aryl group, or a substituted or unsubstituted C₄-C₁₈ heteroaryl group.
Embodiment 3: The method of embodiment 1, wherein the hydrocarbyl group is a substituted or unsubstituted C₁-C₁₆ alkyl group, a substituted or unsubstituted C₃-C₁₆ cycloalkyl group, a substituted or unsubstituted C₃-C₁₆ heterocycloalkyl group, a substituted or unsubstituted C₆-C₁₆ aryl group, a substituted or unsubstituted C₄-C₁₆ heteroaryl group, or a combination comprising at least one of the foregoing.
Embodiment 4: The method of embodiment 1, wherein the hydrocarbyl group is a substituted or unsubstituted C₁-C₁₂ alkyl group, preferably an unsubstituted C₂-C₈ alkyl group, more preferably an unsubstituted C₃-C₈ alkyl group.
Embodiment 5: The method of one or more of embodiments 1 to 4, wherein providing the epoxidized elastomer comprises epoxidizing an unsaturated elastomer to form the epoxidized elastomer.
Embodiment 6: The method of embodiment 5, wherein the unsaturated elastomer comprises at least 0.1 mole% unsaturation, preferably 0.1 to 60 mole% unsaturation, more preferably 0.1 to 40 mole% unsaturation.
Embodiment 7: The method of any one or more of embodiments 5 to 6, wherein the unsaturated elastomer comprises a natural polyisoprene rubber, a synthetic polyisoprene rubber, a homopolymer of 1,3-butadiene, 2-methyl-1,3-butadiene, 1,3-pentadiene, 2-chloro-1,3 butadiene, or 2,3-dimethyl-1,3-butadiene, a copolymer of 1,3-butadiene, 2-methyl-1,3-butadiene, 1,3-pentadiene, 2-chloro-1,3 butadiene, or 2,3-dimethyl-1,3-butadiene with styrene, alpha-methylstyrene, acrylonitrile, isoprene, methacrylonitrile, methyl acrylate, ethyl acrylate, methyl methacrylate, ethyl methacrylate, or vinyl acetate, chloroprene rubber, butyl rubber, halogenated butyl rubber, nitrile rubber, hydrogenated nitrile rubber, ethylene-propylene rubber, ethylene-propylene-diene rubber, unsaturated silicone rubber, or a combination comprising at least one of the foregoing.
Embodiment 8: The method of any one or more of embodiments 5 to 6, wherein the unsaturated elastomer comprises polybutadiene rubber, poly(acrylonitrilebutadiene), poly(styrene-butadiene) rubber, polyisoprene rubber, neoprene rubber, preferably polybutadiene rubber, poly(styrene-butadiene) rubber, or a combination comprising at least one of the foregoing.
Embodiment 9: The method of any one or more of embodiments 5 to 8, wherein the epoxidation is in the presence of peroxide, preferably a peroxyacid, more preferably peroxybenzoic acid, most preferably m-chloroperoxybenzoic acid.
Embodiment 10: The method of any one or more of embodiments 5 to 9, wherein the epoxidation is in a solvent, preferably an aliphatic, cycloaliphatic, heterocycloaliphatic, aromatic, or heteroaromatic solvent, more preferably dichloromethane, chloroform, n-hexane, cyclohexane, n-heptane, benzene, toluene, tetrahydrofuran, or a combination comprising at least one of the foregoing.
Embodiment 11: The method of embodiment 10, wherein the elastomer has a concentration in the solvent of 1 to 22% (w/v), preferably from 2 to 16% (w/v).
Embodiment 12: The method of one or more of embodiments 5 to 11, further comprising quenching the epoxidation by addition of a an aqueous or alcoholic base, preferably an aqueous solution of sodium carbonate or sodium bicarbonate, or a methanolic solution of tetrabutylammonium hydroxide, or potassium hydroxide, or a combination comprising at least one of the foregoing.
Embodiment 13: The method of any one or more of embodiments 1 to 12, wherein the epoxidized elastomer has a degree of epoxidation of 1 to 50 mole percent, preferably 2 to 40 mole percent, more preferably 3 to 30 mole percent.
Embodiment 14: The method of any one or more of embodiments 5 to 13, wherein the epoxidation and the epoxy ring-opening is conducted without isolation of the epoxidized elastomer.
Embodiment 15: The method of any one or more of embodiments 1 to 14, wherein the epoxy-ring opening is in the presence of a base.
Embodiment 16: The method of embodiment 15, wherein the base is an alkali earth or alkaline earth oxide or hydroxide, a quaternized tetra(C₁-C₁₂ hydrocarbyl)ammonium or tetra(C₁-C₁₂ hydrocarbyl)phosphonium salt, or a combination comprising at least one of the foregoing.
Embodiment 17: The method of embodiment 16, wherein the base is tetrabutylammonium hydroxide, potassium hydroxide, or a combination comprising at least one of the foregoing.
Embodiment 18: The method of any one or more of embodiments 1 to 17, wherein the thiol has a concentration of 5 to 70 weight percent, preferably 10 to 60 weight percent, more preferably 15 to 50 weight percent, based on the weight of the epoxidized elastomer.
Embodiment 19: The method of any one or more of embodiments 1 to 18, wherein the epoxy ring-opening is in the presence of a solvent, preferably an aliphatic, cycloaliphatic, heterocycloaliphatic, aromatic, heteroaromatic solvent, more preferably dichloromethane, chloroform, n-hexane, cyclohexane, n-heptane, benzene, toluene, tetrahydrofuran, or a combination comprising at least one of the foregoing.
Embodiment 20: The method of embodiment 19, wherein the epoxidized elastomer has a concentration in the solvent of 1 to 22 % (w/v), preferably from 2 to 16 % (w/v).
Embodiment 21: The method of any one or more of embodiments 1 to 20, wherein the epoxy ring-opening is conducted at a temperature of 0 to 100°C, preferably of 20 to 80°C, more preferably 40 to 60°C.
Embodiment 22: The functionalized elastomer prepared by the method of any one or more of embodiments 1 to 21.
Embodiment 23: A functionalized elastomer comprising an epoxy functional group, a hydroxy functional group, and a C₁-C₃₂ hydrocarbyl-substituted thio functional group, wherein the hydroxy functional group and the C₁-C₃₂ hydrocarbyl-substituted thio functional group are vicinal functional groups.
Embodiment 24: The elastomer of embodiment 23, wherein hydrocarbyl group is a substituted or unsubstituted C₁-C₃₂ alkyl group, a substituted or unsubstituted C₂-C₃₂ alkenyl group, a substituted or unsubstituted C₃-C₁₈ cycloalkyl group, a substituted or unsubstituted C₃-C₁₈ heterocycloalkyl group, a substituted or unsubstituted C₆-C₁₈ aryl group, or a substituted or unsubstituted C₄-C₁₈ heteroaryl group.
Embodiment 25: The elastomer of embodiment 23, wherein the hydrocarbyl group is a substituted or unsubstituted C₁-C₁₆ alkyl group, a substituted or unsubstituted C₃-C₁₆ cycloalkyl group, a substituted or unsubstituted C₃-C₁₆ heterocycloalkyl group, a substituted or unsubstituted C₆-C₁₆ aryl group, a substituted or unsubstituted C₄-C₁₆ heteroaryl group, or a combination comprising at least one of the foregoing.
Embodiment 26: The elastomer of embodiment 23, wherein the hydrocarbyl group is substituted or unsubstituted C₁-C₁₂ alkyl group, preferably an unsubstituted C₂-C₈ alkyl group, more preferably an unsubstituted C₃-C₈ alkyl group,.
Embodiment 27: The elastomer of any one or more of embodiments 23 to 26, wherein the elastomer has a total hydroxy, epoxy, and thio functionalization of 1 to 15 mole percent, preferably 1 to 12 mole percent, more preferably 1 to 10 mole percent.
Embodiment 28: The elastomer of any one or more of embodiments 23 to 27, wherein the elastomer further comprises unsaturation.
Embodiment 29: An article comprising the elastomer of any one or more of embodiments 22 to 28.
Embodiment 30: The article of embodiment 29, wherein the article is a tire tread.

The singular forms "a," "an," and "the" include plural referents unless the context clearly dictates otherwise. "Or" means "and/or." The endpoints of all ranges directed to the same component or property are inclusive and independently combinable. Disclosure of a narrower range or more specific group in addition to a broader range is not a disclaimer of the broader range or larger group. Unless defined otherwise, technical and scientific terms used herein have the same meaning as is commonly understood by one of skill in the art to which this invention belongs. A "combination" is inclusive of blends, mixtures, alloys, reaction products, and the like.

All cited patents, patent applications, and other references are incorporated herein by reference in their entirety. However, if a term in the present application contradicts or conflicts with a term in the incorporated reference, the term from the present application takes precedence over the conflicting term from the incorporated reference.

As used herein, the term "hydrocarbyl" includes groups containing carbon, hydrogen, and optionally one or more heteroatoms (e.g., 1, 2, 3, or 4 atoms such as halogen, O, N, S, P, or Si), which can be present as one or more functional groups. "Alkyl" means a branched or straight chain, saturated, monovalent hydrocarbon group, e.g., methyl, ethyl, i-propyl, and n-butyl. "Alkylene" means a straight or branched chain, saturated, divalent hydrocarbon group (e.g., methylene (-CH₂-) or propylene (-(CH₂)₃-)). "Alkenyl" and "alkenylene" mean a monovalent or divalent, respectively, straight or branched chain hydrocarbon group having at least one carbon-carbon double bond (e.g., ethenyl (-HC=CH₂) or propenylene (-HC(CH₃)=CH₂-). "Alkynyl" means a straight or branched chain, monovalent hydrocarbon group having at least one carbon-carbon triple bond (e.g., ethynyl). "Alkoxy" means an alkyl group linked via an oxygen (i.e., alkyl-O-), for example methoxy, ethoxy, and sec-butyloxy. "Cycloalkyl" and "cycloalkylene" mean a monovalent and divalent cyclic hydrocarbon group, respectively, of the formula -CₙH₂ₙ₋ₓ and -CₙH₂ₙ₋₂ₓ₋wherein x is the number of cyclization(s). "Aryl" means a monovalent, monocyclic or polycyclic, aromatic group (e.g., phenyl or naphthyl). "Arylene" means a divalent, monocyclic or polycyclic, aromatic group (e.g., phenylene or naphthylene). The prefix "halo" means a group or compound including one more halogen (F, Cl, Br, or I) substituents, which can be the same or different. The prefix "hetero" means a group or compound that includes at least one ring member that is a heteroatom (e.g., 1, 2, or 3) heteroatoms, wherein each heteroatom is independently N, O, S, or P.

"Substituted" means that the compound or group is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g, benzyl), C₇₋₁₂ alkylarylene (e.g, toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-), provided that the substituted atom's normal valence is not exceeded, and that the substitution does not significantly adversely affect the manufacture, stability, or desired property of the compound. When a compound is substituted, the indicated number of carbon atoms is the total number of carbon atoms in the group, including those of the substituent(s).

## Claims

1. A method of preparing a functionalized elastomer, the method comprising:
providing an epoxidized elastomer;
epoxy ring-opening of the epoxidized elastomer with a C₁-C₃₂ hydrocarbyl-substituted thiol to provide a functionalized elastomer comprising:
an epoxy functional group,
a hydroxy functional group, and
a substituted or unsubstituted C₁-C₃₂ hydrocarbyl-substituted thio functional group,
wherein the hydroxy functional group and the substituted or unsubstituted C₁-C₃₂ hydrocarbyl-substituted thio functional group are vicinal functional groups.

2. The method of claim 1, wherein the hydrocarbyl group is a substituted or unsubstituted C₁-C₁₆ alkyl group, a substituted or unsubstituted C₃-C₁₆ cycloalkyl group, a substituted or unsubstituted C₃-C₁₆ heterocycloalkyl group, a substituted or unsubstituted C₆-C₁₆ aryl group, a substituted or unsubstituted C₄-C₁₆ heteroaryl group, or a combination comprising at least one of the foregoing, preferably an unsubstituted C₃-C₈ alkyl group.

3. The method of one or more of claims 1 to 2, wherein providing the epoxidized elastomer comprises epoxidizing an unsaturated elastomer to form the epoxidized elastomer.

4. The method of claim 3, wherein the unsaturated elastomer comprises a natural polyisoprene rubber, a synthetic polyisoprene rubber, a homopolymer of 1,3-butadiene, 2-methyl-1,3-butadiene, 1,3-pentadiene, 2-chloro-1,3 butadiene, or 2,3-dimethyl-1,3-butadiene, a copolymer of 1,3-butadiene, 2-methyl-1,3-butadiene, 1,3-pentadiene, 2-chloro-1,3 butadiene, or 2,3-dimethyl-1,3-butadiene with styrene, alpha-methylstyrene, acrylonitrile, isoprene, methacrylonitrile, methyl acrylate, ethyl acrylate, methyl methacrylate, ethyl methacrylate, or vinyl acetate, chloroprene rubber, butyl rubber, halogenated butyl rubber, nitrile rubber, hydrogenated nitrile rubber, ethylene-propylene rubber, ethylene-propylene-diene rubber, unsaturated silicone rubber, or a combination comprising at least one of the foregoing, preferably wherein the unsaturated elastomer comprises polybutadiene rubber, poly(acrylonitrile-butadiene), poly(styrene-butadiene) rubber, polyisoprene rubber, neoprene rubber, preferably polybutadiene rubber, poly(styrene-butadiene) rubber, or a combination comprising at least one of the foregoing.

5. The method of any one or more of claims 3 to 4, wherein epoxidizing is in the presence of peroxide, preferably a peroxyacid, more preferably peroxybenzoic acid, most preferably m-chloroperoxybenzoic acid.

6. The method of any one or more of claims 3 to 5, wherein the epoxidation is in a solvent, preferably an aliphatic, cycloaliphatic, heterocycloaliphatic, aromatic, or heteroaromatic solvent, more preferably dichloromethane, chloroform, n-hexane, cyclohexane, n-heptane, benzene, toluene, tetrahydrofuran, or a combination comprising at least one of the foregoing, the elastomer having a concentration in the solvent of 1 to 22% (w/v), preferably from 2 to 16% (w/v).

7. The method of one or more of claims 3 to 6, further comprising quenching the epoxidation by addition of a an aqueous or alcoholic base, preferably an aqueous solution of sodium carbonate or sodium bicarbonate, or a methanolic solution of tetrabutylammonium hydroxide, or potassium hydroxide, or a combination comprising at least one of the foregoing.

8. The method of any one or more of claims 1 to 7, wherein the epoxidized elastomer has a degree of epoxidation of 1 to 50 mole percent, preferably 2 to 40 mole percent, more preferably 3 to 30 mole percent.

9. The method of any one or more of claims 1 to 8, wherein the epoxy-ring opening is in the presence of a base, the base being an alkali earth or alkaline earth oxide or hydroxide, a quaternized tetra(C₁-C₁₂ hydrocarbyl)ammonium or tetra(C₁-C₁₂ hydrocarbyl)phosphonium salt, or a combination comprising at least one of the foregoing, preferably tetrabutylammonium hydroxide and/or potassium hydroxide.

10. The method according to any one of claims 1 to 9 wherein the hydrocarbyl-substituted thiol is selected from 1-propanethiol, 1-butanethiol, 2-methyl-1-propanethiol, 1-pentanethiol, 3-methyl-1-butanethiol, 1-hexanethiol, 1-heptanethiol, 1-octanethiol, 2-ethylhexanethiol, cyclopentanethiol, cyclohexanethiol, 2-phenylethanethiol, phenylthiol, 2-propene-1-thiol, 2-furanemethanethiol, or combinations thereof.

11. The method of any one or more of claims 1 to 10, wherein the epoxy ring-opening is in the presence of a solvent, preferably an aliphatic, cycloaliphatic, heterocycloaliphatic, aromatic, heteroaromatic solvent, more preferably dichloromethane, chloroform, n-hexane, cyclohexane, n-heptane, benzene, toluene, tetrahydrofuran, or a combination comprising at least one of the foregoing, the epoxidized elastomer having a concentration in the solvent of 1 to 22 % (w/v), preferably from 2 to 16 % (w/v).

12. The method of any one or more of claims 1 to 11, wherein the epoxy ring-opening is conducted at a temperature of 0 to 100°C, preferably of 20 to 80°C, more preferably 40 to 60°C.

13. A functionalized elastomer comprising:
an epoxy functional group,
a hydroxy functional group, and
a C₁-C₃₂ hydrocarbyl-substituted thio functional group,
wherein the hydroxy functional group and the C₁-C₃₂ hydrocarbyl-substituted thio functional group are vicinal functional groups, and wherein the hydrocarbyl group is an substituted or unsubstituted C₁-C₁₂ alkyl group, preferably an unsubstituted C₂-C₈ alkyl group, more preferably an unsubstituted C₃-C₈ alkyl group.

14. An article comprising the functional elastomer according claim 13 or produced according to the method of any one of claims 1-12, wherein the article is a tire tread.

## Patentansprüche

1. Verfahren zur Herstellung eines funktionalisierten Elastomers, das Folgendes umfasst:
Bereitstellen eines epoxidierten Elastomers;
Epoxidringöffnung des epoxidierten Elastomers mit einem C₁-C₃₂-hydrocarbylsubstituierten Thiol zur Bereitstellung eines funktionalisierten Elastomers mit:
einer epoxidfunktionellen Gruppe,
einer hydroxyfunktionellen Gruppe und
einer substituierten oder unsubstituierten C₁-C₃₂-hydrocarbylsubstituierten thiofunktionellen Gruppe,
wobei es sich bei der hydroxyfunktionellen Gruppe und der substituierten oder unsubstituierten C₁-C₃₂-hydrocarbylsubstituierten thiofunktionellen Gruppe um vicinale funktionelle Gruppen handelt.

2. Verfahren nach Anspruch 1, wobei es sich bei der Hydrocarbylgruppe um eine substituierte oder unsubstituierte C₁-C₁₆-Alkylgruppe, eine substituierte oder unsubstituierte C₃-C₁₆-Cycloalkylgruppe, eine substituierte oder unsubstituierte C₃-C₁₆-Heterocycloalkylgruppe, eine substituierte oder unsubstituierte C₆-C₁₆-Arylgruppe, eine substituierte oder unsubstituierte C₄-C₁₆-Heteroaryl-gruppe oder eine Kombination, die mindestens eine der vorstehenden Gruppen umfasst, vorzugsweise eine unsubstituierte C₃-C₈-Alkylgruppe, handelt.

3. Verfahren nach einem oder mehreren der Ansprüche 1 bis 2, wobei das Bereitstellen des epoxidierten Elastomers das Epoxidieren eines ungesättigten Elastomers zu dem epoxidierten Elastomer umfasst.

4. Verfahren nach Anspruch 3, wobei das ungesättigte Elastomer einen natürlichen Polyisoprenkautschuk, einen synthetischen Polyisoprenkautschuk, ein Homopolymer von 1,3-Butadien, 2-Methyl-1,3-butadien, 1,3-Pentadien, 2-Chlor-1,3-butadien oder 2,3-Dimethyl-1,3-butadien, ein Copolymer von 1,3-Butadien, 2-Methyl-1,3-butadien, 1,3-Pentadien, 2-Chlor-1,3-butadien oder 2,3-Dimethyl-1,3-butadien mit Styrol, alpha-Methylstyrol, Acrylnitril, Isopren, Methacrylnitril, Methylacrylat, Ethylacrylat, Methylmethacrylat, Ethylmethacrylat oder Vinylacetat, Chloroprenkautschuk, Butylkautschuk, halogenierten Butylkautschuk, Nitrilkautschuk, hydrierten Nitrilkautschuk, Ethylen-Propylen-Kautschuk, Ethylen-Propylen-Dien-Kautschuk, ungesättigten Silikonkautschuk oder eine Kombination, die mindestens eines der vorstehenden Elastomere umfasst, vorzugsweise wobei das ungesättigte Elastomer Polybutadienkautschuk, Poly(acrylnitril-butadien), Poly(styrol-butadien)-Kautschuk, Polyisoprenkautschuk, Neoprenkautschuk, vorzugsweise Polybutadienkautschuk, Poly(acrylnitril-butadien), oder eine Kombination, die mindestens eines der vorstehenden Elastomere umfasst, umfasst.

5. Verfahren nach einem oder mehreren der Ansprüche 3 bis 4, wobei die Epoxidation in Gegenwart von Peroxid, vorzugsweise einer Peroxysäure, weiter bevorzugt Peroxybenzoesäure, ganz besonders bevorzugt m-Chlorperoxybenzoesäure, erfolgt.

6. Verfahren nach einem oder mehreren der Ansprüche 3 bis 5, wobei die Epoxidation in einem Lösungsmittel, vorzugsweise einem aliphatischen, cycloaliphatischen, heterocycloaliphatischen, aromatischen oder heteroaromatischen Lösungsmittel, weiter bevorzugt Dichlormethan, Chloroform, n-Hexan, Cyclohexan, n-Heptan, Benzol, Toluol, Tetrahydrofuran oder einer Kombination, die mindestens eines der vorstehenden Lösungsmittel umfasst, erfolgt, wobei das Elastomer eine Konzentration in dem Lösungsmittel von 1 bis 22 % (w/v), vorzugsweise von 2 bis 16 % (w/v), aufweist.

7. Verfahren nach einem oder mehreren der Ansprüche 3 bis 6, ferner umfassend das Quenchen der Epoxidation durch Zugabe einer wässrigen oder alkoholischen Base, vorzugsweise einer wässrigen Lösung von Natriumcarbonat oder Natriumhydrogencarbonat oder einer methanolischen Lösung von Tetrabutylammoniumhydroxid oder Kaliumhydroxid oder einer Kombination, die mindestens eine der vorstehenden Basen umfasst.

8. Verfahren nach einem oder mehreren der Ansprüche 1 bis 7, wobei das epoxidierte Elastomer einen Epoxidationsgrad von 1 bis 50 Molprozent, vorzugsweise 2 bis 40 Molprozent, weiter bevorzugt 3 bis 30 Molprozent, aufweist.

9. Verfahren nach einem oder mehreren der Ansprüche 1 bis 8, bei dem die Epoxidringöffnung in Gegenwart einer Base erfolgt, wobei es sich bei der Base um ein Alkali- oder Erdalkalioxid oder -hydroxid, ein quaternisiertes Tetra (C1-C12-hydrocarbyl) ammonium- oder Tetra (C₁-C₁₂-hydrocarbyl)phosphoniumsalz oder eine Kombination, die mindestens eine der vorstehenden Basen umfasst, vorzugsweise Tetrabutylammoniumhydroxid und/oder Kaliumhydroxid, handelt.

10. Verfahren nach einem der Ansprüche 1 bis 9, bei dem das hydrocarbylsubstituierte Thiol aus 1-Propanthiol, 1-Butanthiol, 2-Methyl-1-propanthiol, 1-Pentanthiol, 3-Methyl-1-butanthiol, 1-Hexanthiol, 1-Heptanthiol, 1-Octanthiol, 2-Ethylhexanthiol, Cyclopentanthiol, Cyclohexanthiol, 2-Phenylethanthiol, Phenylthiol, 2-Propen-1-thiol, 2-Furanmethanthiol oder Kombinationen davon ausgewählt wird.

11. Verfahren nach einem oder mehreren der Ansprüche 1 bis 10, wobei die Epoxidringöffnung in Gegenwart eines Lösungsmittels vorzugsweise eines aliphatischen, cycloaliphatischen, heterocycloaliphatischen, aromatischen, heteroaromatischen Lösungsmittels, weiter bevorzugt Dichlormethan, Chloroform, n-Hexan, Cyclohexan, n-Heptan, Benzol, Toluol, Tetrahydrofuran oder einer Kombination, die mindestens eines der vorstehenden Lösungsmittel umfasst, erfolgt, wobei das epoxidierte Elastomer eine Konzentration in dem Lösungsmittel von 1 bis 22 % (w/v), vorzugsweise von 2 bis 16 % (w/v), aufweist.

12. Verfahren nach einem oder mehreren der Ansprüche 1 bis 11, wobei die Epoxidringöffnung bei einer Temperatur von 0 bis 100 °C, vorzugsweise 20 bis 80 °C, weiter bevorzugt 40 bis 60 °C, durchgeführt wird.

13. Funktionalisiertes Elastomer mit:
einer epoxidfunktionellen Gruppe,
einer hydroxyfunktionellen Gruppe und
einer C₁-C₃₂-hydrocarbylsubstituierten thiofunktionellen Gruppe,
wobei es sich bei der hydroxyfunktionellen Gruppe und der C₁-C₃₂-hydrocarbylsubstituierten thiofunktionellen Gruppe um vicinale funktionelle Gruppen handelt und wobei es sich bei der Hydrocarbylgruppe um eine substituierte oder unsubstituierte C₁-C₁₂-Alkylgruppe, vorzugsweise eine unsubstituierte C₂-C₈-Alkylgruppe, weiter bevorzugt eine unsubstituierte C₃-C₈-Alkylgruppe, handelt.

14. Artikel, umfassend das funktionalisierte Elastomer nach Anspruch 13 oder das gemäß dem Verfahren nach einem der Ansprüche 1-12 hergestellte funktionalisierte Elastomer, wobei es sich bei dem Artikel um eine Reifenlauffläche handelt.

## Revendications

1. Procédé de fabrication d'un élastomère fonctionnalisé, le procédé comprenant :
la fourniture d'un élastomère époxydé ;
l'ouverture du cycle époxy de l'élastomère époxydé avec un thiol substitué avec un groupe hydrocarbyle C₁-C₃₂ pour produire un élastomère fonctionnalisé comprenant :
un groupe fonctionnel époxy,
un groupe fonctionnel hydroxy, et
un groupe fonctionnel thio substitué avec un groupe hydrocarbyle C₁-C₃₂ substitué ou non substitué,
dans lequel le groupe fonctionnel hydroxy et le groupe fonctionnel thio substitué avec un groupe hydrocarbyle C₁-C₃₂ substitué ou non substitué sont des groupes fonctionnels vicinaux.

2. Procédé selon la revendication 1, dans lequel le groupe hydrocarbyle est un groupe alkyle C₁-C₁₆ substitué ou non substitué, un groupe cycloalkyle C₃-C₁₆ substitué ou non substitué, un groupe hétérocycloalkyle C₃-C₁₆ substitué ou non substitué, un groupe aryle C₆-C₁₆ substitué ou non substitué, un groupe hétéroaryle C₄-C₁₆ substitué ou non substitué, ou une combinaison comprenant au moins l'un des groupes ci-dessus, préférablement un groupe alkyle C₃-C₈ non substitué.

3. Procédé selon une ou plusieurs des revendications 1 ou 2, dans lequel la fourniture de l'élastomère époxydé comprend l'époxydation d'un élastomère insaturé pour former l'élastomère époxydé.

4. Procédé selon la revendication 3, dans lequel l'élastomère insaturé comprend un caoutchouc polyisoprène naturel, un caoutchouc polyisoprène synthétique, un homopolymère de 1,3-butadiène, de 2-méthyl-1,3-butadiène, de 1,3-pentadiène, de 2-chloro-1,3 butadiène, ou de 2,3-diméthyl-1,3-butadiène, un copolymère de 1,3-butadiène, de 2-méthyl-1,3-butadiène, de 1,3-pentadiène, de 2-chloro-1,3-butadiène ou de 2,3-diméthyl-1,3-butadiène avec du styrène, de l'alphaméthylstyrène, de l'acrylonitrile, de l'isoprène, du méthacrylonitrile, de l'acrylate de méthyle, de l'acrylate d'éthyle, du méthacrylate de méthyle, du méthacrylate d'éthyle, ou de l'acétate de vinyle, un caoutchouc chloroprène, un caoutchouc butyle, un caoutchouc butyle halogéné, un caoutchouc nitrile, un caoutchouc nitrile hydrogéné, un caoutchouc éthylène-propylène, un caoutchouc éthylène-propylène-diène, un caoutchouc silicone insaturé, ou une combinaison comprenant au moins l'un des composés ci-dessus, préférablement dans lequel l'élastomère insaturé comprend un caoutchouc polybutadiène, un poly(acrylonitrile-butadiène), un caoutchouc poly(styrène-butadiène), un caoutchouc polyisoprène, un caoutchouc néoprène, préférablement un caoutchouc polybutadiène, un caoutchouc poly(styrène-butadiène), ou une combinaison comprenant au moins l'un des composés ci-dessus.

5. Procédé selon une ou plusieurs quelconques des revendications 3 à 4, dans lequel l'époxydation a lieu en présence d'un peroxyde, préférablement un peroxyacide, plus préférablement l'acide peroxybenzoïque, idéalement l'acide m-chloroperoxybenzoïque.

6. Procédé selon une ou plusieurs quelconques des revendications 3 à 5, dans lequel l'époxydation a lieu dans un solvant, préférablement un solvant aliphatique, cycloaliphatique, hétérocycloaliphatique, aromatique, ou hétéroaromatique, plus préférablement le dichlorométhane, le chloroforme, le n-hexane, le cyclohexane, le n-heptane, le benzène, le toluène, le tétrahydrofurane, ou une combinaison comprenant au moins l'un des solvants ci-dessus, l'élastomère ayant une concentration dans le solvant de 1 à 22 % (p/v), préférablement de 2 à 16 % (p/v).

7. Procédé selon une ou plusieurs des revendications 3 à 6, comprenant en outre une interruption de l'époxydation par addition d'une base aqueuse ou alcoolique, préférablement une solution aqueuse de carbonate de sodium ou de bicarbonate de sodium, ou une solution méthanolique d'hydroxyde de tétrabutylammonium ou d'hydroxyde de potassium, ou une combinaison comprenant au moins l'une des bases ci-dessus.

8. Procédé selon une ou plusieurs quelconques des revendications 1 à 7, dans lequel l'élastomère époxydé a un degré d'époxydation de 1 à 50 %mol, préférablement de 2 à 40 %mol, plus préférablement de 3 à 30 %mol.

9. Procédé selon une ou plusieurs quelconques des revendications 1 à 8, dans lequel l'ouverture du cycle époxy a lieu en présence d'une base, la base étant un oxyde ou un hydroxyde alcalin ou alcalino-terreux, un sel de tétra(hydrocarbyle C₁-C₁₂) ammonium ou de tétra(hydrocarbyle C₁-C₁₂)phosphonium quaternisé, ou une combinaison comprenant au moins l'une des bases ci-dessus, préférablement de l'hydroxyde de tétrabutylammonium et/ou de l'hydroxyde de potassium.

10. Procédé selon l'une quelconque des revendications 1 à 9, dans lequel le thiol substitué avec un groupe hydrocarbyle est sélectionné parmi le 1-propanéthiol, le 1-butanéthiol, le 2-méthyl-1-propanéthiol, le 1-pentanéthiol, le 3-méthyl-1-butanéthiol, le 1-hexanéthiol, le 1-heptanéthiol, le 1-octanéthiol, le 2-éthylhexanéthiol, le cyclopentanéthiol, le cyclohexanéthiol, le 2-phényléthanéthiol, le phénylthiol, le 2-propène-1-thiol, le 2-furaneméthanéthiol, ou des combinaisons de ceux-ci.

11. Procédé selon une ou plusieurs quelconques des revendications 1 à 10, dans lequel l'ouverture du cycle époxy a lieu en présence d'un solvant, préférablement un solvant aliphatique, cycloaliphatique, hétérocycloaliphatique, aromatique, hétéroaromatique, plus préférablement le dichlorométhane, le chloroforme, le n-hexane, le cyclohexane, le n-heptane, le benzène, le toluène, le tétrahydrofurane, ou une combinaison comprenant au moins l'un des solvants ci-dessus, l'élastomère époxydé ayant une concentration dans le solvant de 1 à 22 % (p/v), préférablement de 2 à 16 % (p/v).

12. Procédé selon une ou plusieurs quelconques des revendications 1 à 11, dans lequel l'ouverture du cycle époxy a lieu à une température de 0 à 100 °C, préférablement de 20 à 80 °C, plus préférablement de 40 à 60 °C.

13. Élastomère fonctionnalisé comprenant :
un groupe fonctionnel époxy,
un groupe fonctionnel hydroxy, et
un groupe fonctionnel thio substitué avec un groupe hydrocarbyle C₁-C₃₂,
dans lequel le groupe fonctionnel hydroxy et le groupe fonctionnel thio substitué avec un groupe hydrocarbyle C₁-C₃₂ sont des groupes fonctionnels vicinaux, et dans lequel le groupe hydrocarbyle est un groupe alkyle C₁-C₁₂ substitué ou non substitué, préférablement un groupe alkyle C₂-C₈ non substitué, plus préférablement un groupe alkyle C₃-C₈ non substitué.

14. Article comprenant l'élastomère fonctionnel selon la revendication 13 ou produit par le procédé selon l'une quelconque des revendications 1 à 12, l'article étant une bande de roulement de pneu.
